# EUROPÄISCHE PATENTANMELDUNG

(11) **EP 4 455 705 A1**
(43) Veröffentlichungstag der Anmeldung: **30.10.2024**
(21) Anmeldenummer: 23170613.6
(22) Anmeldetag: 28.04.2023
(51) Int. Cl.: G01R 33/34, G01R 33/341

(54) **LOKALSPULE UND MAGNETRESONANZVORRICHTUNG**

(71) Anmelder: Siemens Healthineers AG, 91301 Forchheim (DE)
(72) Erfinder: GREISER, Andreas, 91054 Erlangen (DE); KEIL, Miriam, 91056 Erlangen (DE); THÖNE, Jens, 91207 Lauf an der Pegnitz (DE); WEBER, Hans, 91054 Erlangen (DE); DENNERT, Sebastian, 91472 Lonnerstadt (DE); HELLINGER, Marion, 91080 Uttenreuth (DE); ROTHARD, Jörg, 96123 Litzendorf (DE); LANZ, Titus, 97084 Würzburg (DE); ODOJ, Florian, 97209 Veitshöchheim (DE); GEISSNER, Marco, 97230 Estenfeld (DE); GRAF, Manuel, 97222 Rimpar (DE)
(74) Vertreter: Siemens Healthineers Patent Attorneys

(57) **Zusammenfassung**

Die Erfindung betrifft eine Lokalspule (26) für eine Magnetresonanzvorrichtung (10), umfassend zumindest eine Antenne (32), ein Basiselement (30), ein Halteelement (31) und ein Führungssystem (33), wobei die zumindest eine Antenne (32) mechanisch mit dem Halteelement (31) verbunden ist, wobei das Haltelement (31) dazu ausgebildet ist, die zumindest eine Antenne (32) in einer anwendungsgemäßen Position an einer diagnostisch relevanten Körperregion eines Patienten (15) zu halten, wobei das Führungssystem (33) mechanisch mit dem Basiselement (30) und dem Halteelement (31) verbunden ist und dazu ausgebildet ist, das Halteelement (31) veränderlich gegenüber dem Basiselement (30) zu positionieren, wobei ein erstes Ende (36) des Halteelements (31) mechanisch mit dem Basiselement (30) verbunden ist und wobei ein dem ersten Ende gegenüberliegendes zweites Ende (37) des Halteelements (31) freischwebend ist. Die Erfindung betrifft ferner eine Magnetresonanzvorrichtung (10), welche dazu ausgebildet ist, mittels einer erfindungsgemäßen Lokalspule (26) Magnetresonanzdaten einer diagnostisch relevanten Körperregion eines Patienten (15) zu erfassen.

## Beschreibung

Unabhängig vom grammatikalischen Geschlecht eines bestimmten Begriffes sind Personen mit männlicher, weiblicher oder anderer Geschlechteridentität mit umfasst.

Erkrankungen der Zähne und des Zahnhalteapparats, wie z. B. Karies oder Parodontitis, werden heutzutage üblicherweise mit röntgenbasierten Bildgebungsverfahren diagnostiziert. Dabei kommen vor allem konventionelle oder digitale Röntgen-Projektionsverfahren, sowie kürzlich auch dreidimensionale Röntgenverfahren, zum Einsatz. Ein Beispiel für ein dreidimensionales Röntgenverfahren stellt die digitale Volumentomographie dar, welche für eine Bildgebung von Zähnen und des Viscerocraniums eingesetzt werden kann.

Ein großer Nachteil von Röntgenverfahren ist die Notwendigkeit des Einsatzes von ionisierender Strahlung für die Bildgebung. Ein Bildgebungsverfahren, welches ionisierende Strahlen vermeidet, stellt die Magnetresonanztomografie dar. Diese ermöglicht typischerweise einen besseren Weichgewebekontrast als Röntgenverfahren und unterstützt standardmäßig eine dreidimensionale Bildgebung eines Untersuchungsobjekts. Weiterhin ermöglicht die Magnetresonanztomografie eine Bildgebung von Zysten sowie eine Erkennung einer Degradation von Dentin, bevor dies durch ein Röntgenverfahren erkennbar wird. Die Magnetresonanztomografie stellt somit eine potenzielle Alternative zu bekannten Röntgenverfahren bei der Bildgebung einer Gebissregion und/oder einer Kieferregion sowie der Diagnose von Zahnerkrankungen dar.

Die Magnetresonanztomografie ist ein bekanntes Bildgebungsverfahren, mit welchem Magnetresonanzbilder eines Inneren des Untersuchungsobjekts erzeugt werden können. Bei der Durchführung einer Magnetresonanzbildgebung wird das Untersuchungsobjekt üblicherweise in einem starken, statischen und homogenen Grundmagnetfeld (BO-Magnetfeld) einer Magnetresonanzvorrichtung positioniert. Das Grundmagnetfeld kann magnetische Feldstärken von 0,2 Tesla bis 7 Tesla aufweisen, sodass sich Kernspins des Untersuchungsobjekts entlang des Grundmagnetfeldes ausrichten. Um sogenannte Kernspinresonanzen auszulösen, werden hochfrequente Signale, sogenannte Anregungsimpulse (B1-Magnetfeld), in das Untersuchungsobjekt eingestrahlt. Jeder Anregungsimpuls bewirkt eine Abweichung einer Magnetisierung bestimmter Kernspins des Untersuchungsobjekts von dem Grundmagnetfeld um einen Betrag, welcher auch als Flipwinkel bekannt ist. Ein Anregungsimpuls kann dabei ein magnetisches Wechselfeld mit einer Frequenz aufweisen, welche der Larmorfrequenz bei der jeweiligen statischen Magnetfeldstärke entspricht. Die angeregten Kernspins können eine rotierende und abklingende Magnetisierung (Kernspinresonanz) aufweisen, welche sich mittels spezieller Antennen als Magnetresonanzsignal erfassen lässt. Zur räumlichen Kodierung der Kernspinresonanzen des Untersuchungsobjekts können dem Grundmagnetfeld magnetische Gradientenfelder überlagert werden.

Die empfangenen Magnetresonanzsignale werden typischerweise digitalisiert und als komplexe Werte in einer k-Raum-Matrix gespeichert. Diese k-Raum-Matrix kann als Grundlage für eine Rekonstruktion von Magnetresonanzbildern sowie eine Bestimmung von Spektroskopiedaten verwendet werden. Die Rekonstruktion eines Magnetresonanzbilds erfolgt typischerweise mittels einer mehrdimensionalen Fourier-Transformation der k-Raum-Matrix.

Derzeit ist eine Anwendung der Magnetresonanztomografie zur Bildgebung von Zähnen und/oder dem Kieferapparat jedoch vor allem auf Forschungsstudien beschränkt. Dies liegt unter anderem daran, dass sich Bildgebungstechniken auf Basis von Röntgenstrahlen rasch durchführen lassen und mit einer zu vernachlässigenden Einschränkung des Patienten verbunden sind. Eine Magnetresonanzuntersuchung kann hingegen mit einem höheren Aufwand hinsichtlich Vorbereitung und Positionierung des Patienten verbunden sein. Weiterhin werden Patienten durch die Lagerung in einem Patiententunnel (z. B. bei geschlossenen oder zylindrischen Magnetresonanzvorrichtungen) oder den Einsatz dedizierter Kopfspulen mitunter stark beeinträchtigt. Dieser Umstand kann insbesondere bei klaustrophobisch veranlagten Patienten oder Kindern eine hohe Hürde darstellen und zu einem vorzeitigen Abbruch einer Magnetresonanzuntersuchung führen.

Es ist daher eine Aufgabe der Erfindung, eine Lokalspule bereitzustellen, welche ein Aufnehmen von Magnetresonanzbildern mit hoher Qualität ermöglicht und eine Einschränkung eines Komforts eines Patienten reduziert.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Die erfindungsgemäße Lokalspule umfasst zumindest eine Antenne, ein Basiselement, ein Halteelement und ein Führungssystem.

Die zumindest eine Antenne ist dazu ausgebildet, hochfrequente Signale in einem Frequenz- und Leistungsbereich einer Magnetresonanzmessung zu empfangen. Eine Antenne kann ein Koppelelement zwischen in Signalleitern geführten und ungeführten, d. h. in einem Freiraum befindlichen, elektromagnetischen Wellen darstellen. Die zumindest eine Antenne ist insbesondere dazu ausgebildet, elektromagnetische Wellen im Bereich einer Magnetresonanzfrequenz eines magnetresonanzaktiven Atomkerns zu empfangen. Für Magnetresonanzmessungen relevante elektromagnetische Wellen können hochfrequente Signale (Magnetresonanzsignale) darstellen, welche Frequenzen zwischen 1 und 500 MHz, vorzugsweise zwischen 10 und 300 MHz, umfassen. Die Magnetresonanzsignale üblicher zu untersuchender Atomkerne können eine geringe Leistung von einigen Mikrowatt bis mehrere Milliwatt aufweisen.

Ein Signalleiter ist vorzugsweise ein elektrisch leitender Draht. Der Draht des Signalleiters kann einen ovalen oder polygonalen Querschnitt aufweisen. Vorzugsweise ist der Draht des Signalleiters dazu ausgebildet, die oben angegebenen Leistungen kontinuierlich zu übertragen. Es ist ebenso vorstellbar, dass der Signalleiter als eine Leiterbahn auf einer Leiterplatte ausgeführt ist. Der Signalleiter kann aus Kupfer bestehen. Es sind aber auch andere elektrisch leitende Metalle, wie z. B. Gold, Aluminium und dergleichen, vorstellbar.

Selbstverständlich kann die erfindungsgemäße Lokalspule mehrere Antennen umfassen. Die Antennen können dabei voneinander beabstandet, aneinander angrenzend oder teilweise überlappend angeordnet sein. Die Antennen können ferner in Form eines Gitters oder einer Matrix angeordnet sein.

In einer bevorzugten Ausführungsform weist die Lokalspule eine Mehrzahl von Antennen auf, welche dazu ausgebildet sind, Magnetresonanzsignale aus einer diagnostisch relevanten Körperregion, insbesondere einer Kopfregion oder einer Kieferregion des Patienten, zu empfangen.

Die zumindest eine Antenne kann mechanisch mit einer Trägerstruktur verbunden sein und von dieser getragen oder gehalten werden. Es ist ebenso vorstellbar, dass die zumindest eine Antenne in die Trägerstruktur integriert oder eingebettet ist. Die Trägerstruktur kann ein Material aufweisen, welches dazu ausgebildet ist, einen Berührschutz für den Patienten bereitzustellen und/oder einer Kontur der diagnostisch relevanten Körperregion des Patienten nachgeformt zu werden.

Es ist vorstellbar, dass die erfindungsgemäße Lokalspule eine Sendeantenne aufweist, welche dazu ausgelegt ist, ein hochfrequentes Signal in eine Richtung des Untersuchungsobjekts, wie z. B. eine Kieferregion des Patienten, auszusenden. Das von der Sendeantenne ausgesandte, hochfrequente Signal kann in Abhängigkeit des Grundmagnetfelds einer Magnetresonanzvorrichtung beispielsweise in einem Leistungsbereich von wenigen Watt bis mehreren Kilowatt liegen. Das von der Sendeantenne ausgesandte, hochfrequente Signal kann insbesondere ein B1-Magnetfeld darstellen. Ein Teil der Lokalspule mit der Sendeantenne kann beispielsweis eine Sendeeinheit der Lokalspule darstellen. Die Sendeantenne kann mit der zumindest einen Antenne übereinstimmen oder getrennt von der zumindest einen Antenne vorliegen.

Die zumindest eine Antenne ist mechanisch mit dem Halteelement verbunden. Vorzugsweise ist das Halteelement dazu ausgebildet, eine Positionierung und/oder Ausrichtung der zumindest einen Antenne gegenüber dem Patienten und/oder dem Basiselement zu ermöglichen. Das Halteelement kann insbesondere dazu ausgebildet sein, die zumindest eine Antenne zu tragen und/oder eine mechanische Unterstützung für einen dritten Führungsmechanismus gemäß einer unten beschriebenen Ausführungsform bereitzustellen.

Das Halteelement ist dazu ausgebildet, die zumindest eine Antenne in einer anwendungsgemäßen Position an einer diagnostisch relevanten Körperregion eines Patienten zu halten.

In einer Ausführungsform ist die zumindest eine Antenne lösbar an dem Haltelement befestigt. Das Halteelement und die zumindest eine Antenne und/oder die Trägerstruktur der zumindest einen Antenne können einen Haltemechanismus aufweisen, welcher dazu ausgebildet ist, die zumindest eine Antenne reversibel mit dem Halteelement mechanisch zu verbinden. Das Haltelement und die zumindest eine Antenne können insbesondere komplementär ausgestaltete Teile des Haltemechanimus aufweisen, welche dazu ausgebildet sind, reversibel ineinander einzugreifen. Beispielsweise kann der Haltemechanismus als Steckverbindung, Schnappverbindung oder Rastverbindung ausgestaltet sein. Daneben sind jedoch auch andere formschlüssige und/oder kraftschlüssige mechanische Verbindungen vorstellbar.

Das Halteelement ist vorzugsweise mit dem Basiselement mechanisch verbunden. Die mechanische Verbindung zwischen dem Halteelement und dem Basiselement kann beispielweise mittels des Führungssystems bereitgestellt sein. Es ist insbesondere vorstellbar, dass die mechanische Verbindung zwischen dem Halteelement und dem Basiselement mittels eines ersten Führungsmechanismus und/oder eines zweiten Führungsmechanismus des Führungssystems bereitgestellt ist. Vorzugsweise sind das Basiselement und das Führungssystem dazu ausgelegt, das Haltelement in einer anwendungsgemäßen Position relativ zu einem durch das Basiselement definierten Aufnahmebereich zu halten.

Ein durch das Basiselement definierter Aufnahmebereich kann ein Volumen und/oder eine Fläche umfassen, in welchem die diagnostisch relevante Körperregion des Patienten für eine Magnetresonanzuntersuchung anwendungsgemäß positioniert ist. Vorzugsweise ist der Aufnahmebereich durch ein an eine Grundfläche des Basiselements angrenzendes Volumen und/oder ein durch das Basiselement eingegrenztes Volumen definiert. Der Aufnahmebereich kann insbesondere durch einen Freiraum charakterisiert sein, welcher zur Aufnahme der diagnostisch relevanten Körperregion des Patienten in der Lokalspule geeignet ist. Die diagnostisch relevante Körperregion des Patienten kann insbesondere ein Abschnitt eines Kopfes oder eine Kieferregion darstellen.

Das Basiselement kann mechanisch mit einer Komponente einer Magnetresonanzvorrichtung, wie z. B. einem Patiententisch und/oder einer Patientenlagerungsvorrichtung, verbunden sein. Weiterhin kann das Basiselement eine Positionierungseinheit aufweisen, welche dazu ausgebildet ist, das Basiselement relativ zu der Magnetresonanzvorrichtung, der Patientenlagerungsvorrichtung und/oder dem Patienten zu positionieren. Das Basiselement kann als eine mechanische Struktur verstanden werden, welche dazu ausgebildet ist, das Haltelement robust und/oder reproduzierbar in der anwendungsgemäßen Position an der diagnostisch relevanten Körperregion des Patienten zu halten. Das Basiselement kann ferner eine mechanische Schnittstelle zwischen der Magnetresonanzvorrichtung und dem Halteelement darstellen.

Das Führungssystem ist mechanisch mit dem Basiselement und dem Halteelement verbunden und dazu ausgebildet, das Halteelement veränderlich gegenüber dem Basiselement zu positionieren.

Das Führungssystem kann einen beliebigen Führungsmechanismus umfassen, welcher ein relatives Bewegen des Halteelements gegenüber dem Basiselement ermöglicht. Vorzugsweise umfasst das Führungssystem ein Gelenk, ein Lager, ein Scharnier, insbesondere einen Schwenk- oder Klappmechanismus, eine Schienenführung, eine Linearführung oder eine Kombination dieser oder vergleichbarer Mechanismen. Das Führungssystem ist vorzugsweise dazu ausgebildet, ein einfaches und/oder zeiteffizientes Überführen der Lokalspule, insbesondere des Halteelements und/oder der zumindest einen Antenne, von einer Öffnungsposition in eine Schließposition zu ermöglichen.

Eine Öffnungsposition der Lokalspule kann durch eine maximale oder vorbestimmte Auslenkung des Halteelements und/oder der zumindest einen Antenne gegenüber einer Auflagefläche der Lokalspule charakterisiert sein. Die Öffnungsposition kann insbesondere eine "Ladeposition" der Lokalspule darstellen, welche ein Positionieren der diagnostisch relevanten Körperregion des Patienten in einer anwendungsgemäßen, relativen Position zu dem Basiselement ermöglicht. Es ist vorstellbar, dass das Haltelement und/oder die zumindest eine Antenne in einer Öffnungsposition der Lokalspule derart positioniert oder angeordnet sind, dass ein Zugang der diagnostisch relevanten Körperregion des Patienten zu dem Aufnahmebereich der Lokalspule ermöglicht wird.

Eine Auflagefläche der Lokalspule kann eine von dem Basiselement eingefasste oder umschlossene Oberfläche der Lokalspule darstellen. Vorzugsweise ist die Auflagefläche dazu ausgebildet, die diagnostisch relevante Körperregion des Patienten in einer anwendungsgemäßen Position für eine Magnetresonanzuntersuchung der diagnostisch relevanten Körperregion aufzunehmen und/oder mechanisch zu stützen. Es ist ebenso vorstellbar, dass die Auflagefläche einen Abschnitt eines Patiententisches oder einer Patientenlagerungsvorrichtung darstellt, welcher von dem Basiselement der Lokalspule umschlossen und/oder eingefasst ist. Insbesondere kann die Auflagefläche eine Fläche in einem Aufnahmebereich der Lokalspule sein, welcher zwischen zwei Begrenzungselementen des Basiselements positioniert oder von dem Basiselement eingefasst ist.

Eine Schließposition der Lokalspule kann durch eine minimale Auslenkung des Haltelements und/oder der zumindest einen Antenne gegenüber der Auflagefläche charakterisiert sein. Die Schließposition kann insbesondere eine "Untersuchungsposition" darstellen, welche durch eine anwendungsgemäße, relative Position der zumindest einen Antenne an der diagnostisch relevanten Körperregion des Patienten für eine Magnetresonanzmessung charakterisiert ist. Ein Überführen der Lokalspule von der Öffnungsposition in die Schließposition kann insbesondere ein Führen des Halteelements und/oder der zumindest einen Antenne in Richtung der Auflagefläche der Lokalspule umfassen. Es ist vorstellbar, dass das Haltelement bei dem Überführen der Lokalspule von der Öffnungsposition in die Schließposition mittels des Führungssystems in einer dem Basiselement zugewandten Richtung bewegt oder ausgelenkt wird.

Erfindungsgemäß ist ein erstes Ende des Halteelements mechanisch mit dem Basiselement verbunden. Ein dem ersten Ende gegenüberliegendes zweites Ende des Halteelements ist freischwebend.

Das erste Ende des Halteelements kann gemäß einer oben beschriebenen Ausführungsform mechanisch mit dem Basiselement verbunden sein.

Vorzugsweise weist die erfindungsgemäße Lokalspule einen Freiraum oder Spalt auf, welcher das zweite Ende des Halteelements von einem dem zweiten Ende des Haltelements nächstgelegenen Punkt auf dem Basiselement trennt. Insbesondere kann der Freiraum oder Spalt das zweite Ende des Halteelements von einem nächstgelegenen Punkt auf einem Begrenzungselement (z. B. einem zweiten Begrenzungselement) des Basiselements trennen. Es ist vorstellbar, dass das Haltelement den Aufnahmebereich in einer X-Richtung der Lokalspule nur entlang eines Abschnitts begrenzt oder einschließt. Vorzugsweise ist der Freiraum dazu ausgebildet, einen Zugang zu dem Aufnahmebereich aus einem Umfeld der Lokalspule bereitzustellen. Der Freiraum ist insbesondere derart ausgestaltet, dass ein Zugang zu dem Aufnahmebereich entlang einer Flächennormalen zu der Auflagefläche ermöglicht wird. Der Freiraum kann eine Abmessung von mehr als 2 cm, mehr als 4 cm oder mehr als 6 cm in einer X-Richtung der Lokalspule aufweisen.

Das Halteelement kann beispielsweise als ein zylindrischer Körper, ein Prisma, ein Arm, ein Rohr, ein Balken oder eine Welle ausgestaltet sein. Vorzugsweise ist ein Durchmesser des Haltelements im Vergleich zu einer Längserstreckung des Halteelements gering. Beispielweise kann der Durchmesser des Halteelements weniger als 10 cm, weniger als 8 cm oder vorzugsweise weniger als 6 cm betragen. Dadurch kann eine Beeinträchtigung einer Sicht des Patienten während einer Magnetresonanzuntersuchung mit der Lokalspule auf vorteilhafte Weise reduziert werden.

Vorzugsweise ist die erfindungsgemäße Lokalspule als eine Dentalspule ausgestaltet, welche dazu ausgebildet ist, Magnetresonanzsignale einer Zahnregion und/oder einer Kieferregion eines Patienten zu erfassen. Das Halteelement kann dazu ausgebildet sein, die zumindest eine Antenne der Lokalspule an einer Mundregion und/oder einer Wangenregion eines Patienten zu halten. Insbesondere kann das Haltelement dazu ausgebildet sein, die zumindest eine Antenne derart in einer anwendungsgemäßen Position an der Mundregion und/oder Wangenregion des Patienten zu halten, dass die zumindest eine Antenne an die Mundregion und/oder die Wangenregion des Patienten angeformt ist und/oder diese zumindest teilweise entlang einer Umfangsrichtung des Kopfes des Patienten umschließt.

Durch ein Halteelement mit einem freischwebenden Ende lässt sich eine offene Lokalspule bereitstellen. Eine offene Lokalspule bietet zahlreiche Vorteile gegenüber konventionellen Lokalspulen, wie z. B. einen verbesserten Zugang zu dem Aufnahmebereich, eine verbesserte Ventilation, eine Vermeidung von Verschattung der diagnostisch relevanten Körperregion, aber auch eine reduzierte Beeinträchtigung eines Sichtfelds des Patienten. Dadurch lässt sich ein Komfort des Patienten während einer Magnetresonanzuntersuchung auf vorteilhafte Weise erhöhen und/oder ein Risiko eines Abbruchs einer Magnetresonanzuntersuchung reduzieren.

In einer Ausführungsform der erfindungsgemäßen Lokalspule weist das Führungssystem einen ersten Führungsmechanismus auf, welcher dazu ausgebildet ist, das Halteelement entlang einer Bogenbahn relativ zu dem Basiselement zu führen.

Eine Bogenbahn kann beispielsweise ein Abschnitt einer Kreisbahn oder einer Ellipsenbahn darstellen.

Der erste Führungsmechanismus kann beispielsweise als ein Lager, ein Gelenk, ein Scharnier oder dergleichen ausgestaltet sein. Es ist vorstellbar, dass der erste Führungsmechanismus dazu ausgebildet ist, ein Schwenken oder Rotieren des Halteelements um einen festen oder beweglichen Punkt, insbesondere einen Lagerpunkt oder einen Achsmittelpunkt, zu ermöglichen.

Der erste Führungsmechanismus kann insbesondere dazu ausgelegt sein, ein Schwenken oder ein Rotieren des Haltelements mit der zumindest einen Antenne um eine X-Achse der Lokalspule zu ermöglichen.

Mittels eines ersten Führungsmechanismus kann eine besonders zeiteffiziente und/oder einfach zu handhabende Überführung der Lokalspule von der Öffnungsposition in die Schließposition realisiert werden. Weiterhin lässt sich ein erfindungsgemäßes Führungssystem auf vorteilhafte Weise auf einer Seite des Basiselements der Lokalspule implementieren, wodurch eine Beeinträchtigung des Patienten durch Komponenten der Lokalspule im Sichtfeld des Patienten auf vorteilhafte Weise reduziert werden kann.

In einer Ausführungsform der erfindungsgemäßen Lokalspule weist das Führungssystem einen zweiten Führungsmechanismus auf, welcher dazu ausgebildet ist, das Halteelement im Wesentlichen entlang einer Flächennormalen einer von dem Basiselement definierten Auflagefläche relativ zu dem Basiselement veränderlich zu positionieren.

Eine Flächennormale der Auflagefläche ist vorzugsweise parallel zu einer Y-Richtung der Lokalspule ausgerichtet. Der zweite Führungsmechanismus kann dazu ausgebildet sein, das Haltelement mit der zumindest einen Antenne unter einer Winkelabweichung von weniger als 30°, weniger als 20° oder weniger als 10° zu der Flächennormalen der Auflagefläche zu bewegen. Der zweite Führungsmechanismus kann insbesondere dazu ausgebildet sein, das Haltelement relativ zu der Auflagefläche und/oder dem Basiselement zu positionieren.

Der zweite Führungsmechanismus kann ein beliebiges Führungselement, wie z. B. eine Schiene, eine Nut, eine Stange, ein Rohr oder dergleichen umfassen. Es ist vorstellbar, dass eine Ausrichtung des Führungselements eine Positionierungsrichtung des Haltelements mit der zumindest einen Antenne definiert.

In einer bevorzugten Ausführungsform sind der erste Führungsmechanismus und der zweite Führungsmechanismus des Führungssystems mechanisch verbunden und/oder integriert. Der erste Führungsmechanismus und der zweite Führungsmechanismus können insbesondere als kombinierter Führungsmechanismus ausgestaltet sein. Beispielsweise ist der zweite Führungsmechanismus dazu ausgebildet, eine Achse oder einen Lagerpunkt des ersten Führungsmechanismus zu verschieben, um einen Bewegungstrajektorie des Halteelements anzupassen oder einzustellen. Es ist jedoch ebenso vorstellbar, dass der erste Führungsmechanismus dazu ausgebildet ist, eine Ausrichtung und/oder Orientierung des zweiten Führungsmechanismus zu verändern, wodurch das Haltelement und/oder die zumindest eine Antenne unter einer Winkelabweichung von weniger als 30°, weniger als 20° oder weniger als 10° zu der Y-Richtung der Lokalspule zu positionierbar ist.

Der erste Führungsmechanismus und der zweite Führungsmechanismus können derart mechanisch verbunden oder integriert sein, dass das Haltelement bei dem Wechsel zwischen Öffnungsposition und Schließposition der Lokalspule nacheinander entlang einer durch den ersten Führungsmechanismus definierten Bewegungstrajektorien und einer durch den zweiten Führungsmechanismus definierten Bewegungstrajektorie führbar ist.

In einer weiteren Ausführungsform sind der Führungsmechanismus und der zweite Führungsmechanismus derart mechanisch verbunden oder integriert, dass eine Bewegungstrajektorie des Halteelements bei einem Wechsel zwischen Öffnungsposition und Schließposition der Lokalspule im Wesentlichen einen Abschnitt einer Ellipsenbahn beschreibt. Der Abschnitt der Ellipsenbahn kann beispielsweise einer kombinierten Bewegungstrajektorie entsprechen, welche sich aus einer Überlagerung einer durch den erstem Führungsmechanismus definierten Bewegungstrajektorien und einer durch den zweiten Führungsmechanismus definierten Bewegungstrajektorie ergibt.

Insbesondere stellen der erste Führungsmechanismus und/oder der zweite Führungsmechanismus eine Führung für das Halteelement bereit. Dies kann bedeuten, dass eine Bewegung des Halteelements mittels des ersten Führungsmechanismus und/oder des zweiten Führungsmechanismus auf eine vorbestimmte Anzahl von Raumrichtungen und/oder Drehrichtungen beschränkt ist. Beispielsweise kann das veränderliche Positionieren des Halteelements gegenüber dem Basiselement mittels des erste Führungsmechanismus auf ein Bewegen des Halteelements entlang einer Kreisbahn beschränkt sein. Das veränderliche Positionieren des Halteelements gegenüber dem Basiselement mittels des zweiten Führungsmechanismus kann dagegen auf ein Bewegen entlang einer Geraden, insbesondere einer Parallelen zu einer Y-Richtung der Lokalspule, beschränkt sein. Durch die mechanische Verbindung oder Integration des ersten Führungsmechanismus und des zweiten Führungsmechanismus kann dagegen ein veränderliches Positionieren des Haltelements und/oder der zumindest einen Antenne entlang einer Ellipsenbahn ermöglicht werden, welche sich aus einer Kombination der Bewegungstrajektorien des ersten Führungsmechanismus und des zweiten Führungsmechanismus ergibt.

Durch das Bereitstellen eines zweiten Führungsmechanismus kann ein zusätzlicher Bewegungsfreiheitsgrad bei einer Positionierung des Halteelements und/oder der zumindest einen Antenne mittels des Führungssystems erhalten werden. Dadurch lässt sich eine Ausrichtung der zumindest einen Antenne relativ zu der diagnostisch relevanten Körperregion des Patienten auf vorteilhafte Weise vereinfachen und/oder beschleunigen.

Durch eine mechanische Verbindung oder Integration des ersten Führungsmechanismus und des zweiten Führungsmechanismus lässt sich ein Volumen oder Bauraum gegenüber einer Ausführungsform mit mechanisch getrennten Führungsmechanismen auf vorteilhafte Weise reduzieren. Dadurch kann eine besonders kompakte Lokalspule bereitgestellt werden, welche offener ist, einen größeren Freiraum bietet und/oder eine Beeinträchtigung des Patienten während der Magnetresonanzuntersuchung reduziert.

In einer Ausführungsform weist die erfindungsgemäße Lokalspule einen Arretierungsmechanismus auf, welcher dazu ausgebildet ist, ein Positionieren des Halteelements mittels des Führungssystems freizugeben und/oder das Führungssystem in einer gewünschten Position zu fixieren.

Der Arretierungsmechanismus kann ein Arretierungselement aufweisen. Vorzugsweise sind der Arretierungsmechanismus und/oder das Arretierungselement mechanisch mit dem ersten Führungsmechanismus und/oder dem zweiten Führungsmechanismus gekoppelt.

Der Arretierungsmechanismus kann dazu ausgebildet sein, eine mittels des ersten Führungsmechanismus und/oder des zweiten Führungsmechanismus auf das Haltelement übertragene Kraft zu unterbrechen, wenn sich das Arretierungselement in einer vorbestimmten relativen Position zu dem ersten Führungsmechanismus und/oder dem zweiten Führungsmechanismus befindet. Das Arretierungselement kann beispielsweise als ein Anschlagselement, wie z. B. ein Stift, ein Bolzen, eine Platte, ein Block, aber auch ein Rastelement, ein Federelement, ein Getriebeelement oder eine Kombination solcher Elemente, ausgestaltet sein.

Es ist vorstellbar, dass der Arretierungsmechanismus dazu ausgebildet ist, eine Bewegung des Halteelements entlang einer durch den ersten Führungsmechanismus und/oder den zweiten Führungsmechanismus vorgegebenen Bewegungstrajektorie freizugeben und/oder zu verhindern.

Der Arretierungsmechanismus kann weiterhin dazu ausgebildet sein, den ersten Führungsmechanismus und/oder den zweiten Führungsmechanismus in einer gewünschten Position zu fixieren. In einer bevorzugten Ausführungsform ist der Arretierungsmechanismus dazu ausgebildet, eine Bewegung des Halteelements entlang der durch den zweiten Führungsmechanismus vorgegebenen Bewegungstrajektorie zu verhindern.

Der Arretierungsmechanismus kann insbesondere dazu ausgebildet sein, eine Bewegung des Halteelements mittels des zweiten Führungsmechanismus zu verhindern, wenn das Arretierungselement in einer Ausgangs-Position vorliegt. Es ist vorstellbar, dass der Arretierungsmechanismus dazu ausgebildet ist, den zweiten Führungsmechanismus durch eine manuelle Krafteinwirkung auf das Arretierungselement durch einen Nutzer freizugeben, sodass das Halteelement entlang der durch den zweiten Führungsmechanismus vorgegebenen Bewegungstrajektorie veränderlich positionierbar ist. Der Arretierungsmechanismus kann beispielsweise ein Federelement aufweisen, welches dazu ausgebildet ist, das Arretierungselement bei Beendigung der Krafteinwirkung durch den Nutzer wieder in die Ausgangs-Position zurückzuführen.

In einer weiteren Ausführungsform ist der Arretierungsmechanismus dazu ausgebildet, ein Positionieren des Halteelements mittels des ersten Führungsmechanismus freizugeben und/oder den ersten Führungsmechanismus in einer gewünschten Position zu fixieren. Der Arretierungsmechanismus kann beispielsweise ein zweites Arretierungselement aufweisen, welches dazu ausgebildet ist, eine Bewegung des Halteelements entlang einer durch den ersten Führungsmechanismus vorgegebenen Bewegungstrajektorie freizugeben und/oder zu verhindern. Es ist ebenso vorstellbar, dass der erste Führungsmechanismus und der zweite Führungsmechanismus mechanisch gekoppelt oder integriert sind. In diesem Fall kann der Arretierungsmechanismus dazu ausgebildet sein, eine Bewegung des Halteelements entlang der durch den ersten Führungsmechanismus und/oder den zweiten Führungsmechanismus vorgegebenen Bewegungstrajektorien freizugeben und/oder zu verhindern.

Durch das Bereitstellen einer erfindungsgemäßen Lokalspule mit einem Arretierungsmechanismus lässt sich ein unbeabsichtigtes Bewegen des Halteelements und/oder der zumindest einen Antenne vermeiden. Dadurch kann ein Risiko einer Kollision mit der diagnostisch relevanten Körperregion des Patienten in dem Aufnahmebereich der Lokalspule auf vorteilhafte Weise verhindert werden.

Eine Kollision kann durch ein ungewünschtes Zusammenstoßen oder Zusammenführen der zumindest einen Antenne mit der diagnostisch relevanten Körperregion des Patienten charakterisiert sein. Eine Kollision kann eine Bewegung der zumindest einen Antenne und/oder des Halteelements in Richtung der diagnostisch relevanten Körperregion des Patienten voraussetzen. Es ist insbesondere vorstellbar, dass die Kollision einen Kontakt, eine Kraftwirkung und/oder eine Kraftübertragung zwischen der einen Antenne und der diagnostisch relevanten Körperregion des Patienten umfasst. Ein Kontakt zwischen der zumindest einen Antenne und der diagnostisch relevanten Körperregion, welcher unter einer Freigabe durch den Arretierungsmechanismus erfolgt, gilt dagegen als ein kontrollierter Kontakt und fällt nicht unter die oben gegebene Definition einer Kollision.

Ein erfindungsgemäßer Arretierungsmechanismus lässt sich auf vorteilhafte Weise mechanisch mit dem Führungssystem koppeln. Dadurch kann ein einseitig freischwebendes Haltelement bereitgestellt werden, welches einen Zugang zu dem Aufnahmebereich der Lokalspule verbessert und/oder eine Beeinträchtigung des Patienten reduziert.

In einer Ausführungsform der erfindungsgemäßen Lokalspule weist der Arretierungsmechanismus ein Adaptionselement auf, welches dazu ausgebildet ist, einen mechanischen Widerstand des Führungssystems veränderlich einzustellen.

Es ist vorstellbar, dass das Adaptionselement ein Spannelement, wie z. B. eine mechanische Feder, einen elastischen Block und/oder einen Zugmechanismus, umfasst. Es ist ebenso vorstellbar, dass das Adaptionselement ein Getriebe mit einer vorbestimmten Kraftübersetzung aufweist. Das Spannelement kann dazu ausgebildet sein, einer Bewegung des Halteelements mittels des ersten Führungsmechanismus und/oder des zweiten Führungsmechanismus eine vorbestimmte Kraft, insbesondere eine elastische Rückstellkraft, entgegenzusetzen. Es ist weiterhin vorstellbar, dass das Adaptionselement dazu ausgebildet ist, einen Reibungswiderstand zwischen Teilen des ersten Führungsmechanismus und/oder Teilen des zweiten Führungsmechanismus einzustellen.

Durch das Bereitstellen einer erfindungsgemäßen Lokalspule mit einem Adaptionselement lässt sich ein Widerstand einer Justierung der zumindest einen Antenne relativ zu der diagnostisch relevanten Körperregion auf vorteilhafte Weise an physische Voraussetzungen eines Nutzers anpassen. Ferner kann das Adaptionselement eine unbeabsichtigte Kollision mit der diagnostischen Körperregion des Patienten verhindern, was bei einer Bedienung der Lokalspule von einer Seite vorteilhaft sein kann.

In einer bevorzugten Ausführungsform weist die erfindungsgemäße Lokalspule einen dritten Führungsmechanismus auf, welcher dazu ausgebildet ist, eine Rotation der zumindest einen Antenne um eine Achse des dritten Führungsmechanismus zu ermöglichen.

Der dritte Führungsmechanismus kann ein Scharnier, ein Gelenk oder ein Lager, insbesondere ein Gleitlager, ein Drehlager, oder ein Wälzlager, aufweisen. In einer bevorzugten Ausführungsform weist der dritte Führungsmechanismus ein Lager auf, welches einen Körper des Halteelements entlang eines Abschnitts außenumfänglich umschließt. Insbesondere kann das Halteelement gemäß einer oben beschriebenen Ausführungsform einen zylindrischen Körper aufweisen. Der dritte Führungsmechanismus kann den zylindrischen Körper des Halteelements entlang eines Zylinderabschnitts außenumfänglich umschließen. Es ist jedoch ebenso vorstellbar, dass der dritte Führungsmechanismus an einem Ende des Halteelements oder zwischen zwei Hälften oder Teilen des Halteelements angeordnet ist. Beispielsweise kann das Halteelement in einer solchen Anordnung als ein Drehlager ausgestaltet sein, welches mittels eines Flansches oder einer vergleichbaren Verbindung mechanisch mit dem Haltelement verbunden ist.

In einer Ausführungsform weist das Haltelement einen zylindrischen Körper auf, wobei der[PP(TIDC1] dritte Führungsmechanismus dazu ausgebildet ist, eine Rotation der zumindest einen Antenne um eine Achse oder eine Symmetrieachse des zylindrischen Körpers des Haltelements zu ermöglichen.

Durch das Bereitstellen eines dritten Führungsmechanismus lässt sich eine Winkelstellung der zumindest einen Antenne relativ zu der Auflagefläche der Lokalspule unabhängig von dem Führungssystem einstellen. Dadurch kann die zumindest eine Antenne auf vorteilhafte Weise aus dem Aufnahmebereich herausgeschwenkt oder -rotiert werden, wodurch eine Offenheit und/oder Zugänglichkeit der Lokalspule weiter verbessert wird.

In einer weiteren Ausführungsform weist die erfindungsgemäße Lokalspule einen zweiten Arretierungsmechanismus auf, welcher dazu ausgebildet ist, eine Rotation der zumindest einen Antenne um die Achse des dritten Führungsmechanismus freizugeben und/oder den dritten Führungsmechanismus in einer gewünschten Position zu fixieren.

Der zweite Arretierungsmechanismus kann ein Arretierungselement und/oder ein Spannelement gemäß einer oben beschriebenen Ausführungsform umfassen. Der zweite Arretierungsmechanismus kann insbesondere dazu ausgebildet sein, einen mechanischen Widerstand bzw. einen Reibungswiderstand des dritten Führungsmechanismus veränderlich einzustellen. Beispielsweise ist der zweite Arretierungsmechanismus dazu ausgebildet, einen erforderlichen Kraftaufwand für eine Bewegung der zumindest einen Antenne entlang einer durch den dritten Führungsmechanismus definierten Bewegungstrajektorie veränderlich einzustellen. In einer bevorzugten Ausführungsform umfasst der zweite Arretierungsmechanismus ein Stellelement, insbesondere eine Stellschraube, welches dazu ausgebildet ist, den mechanischen Widerstand des dritten Führungsmechanismus kontinuierlich oder in diskreten Schritten einzustellen. Das Stellelement kann mechanisch mit einem Spannelement gekoppelt sein. Ein Stellelement kann es dem Nutzer auf vorteilhafte Weise ermöglichen, eine Einstellung des Arretierungsmechanismus vorzunehmen.

Der zweite Arretierungsmechanismus ist insbesondere dazu ausgelegt, eine Relativbewegung zwischen dem Halteelement und der zumindest einen Antenne zu verhindern oder freizugeben.

Durch das Bereitstellen einer erfindungsgemäßen Lokalspule mit einem zweiten Arretierungsmechanismus lässt sich auf vorteilhafte Weise eine besonders einfach zu bedienende und/oder kompakte Möglichkeit zur Positionierung der zumindest einen Antenne relativ zu der diagnostisch relevanten Körperregion des Patienten realisieren. Dadurch lässt sich eine Beeinträchtigung des Patienten reduzieren und/oder eine Bedienung der erfindungsgemäßen Lokalspule von einer Seite vereinfachen.

In einer Ausführungsform weist die erfindungsgemäße Lokalspule einen vierten Führungsmechanismus auf. Der vierte Führungsmechanismus ist mechanisch mit dem Haltelement verbunden und dazu ausgebildet, ein Hilfselement entlang einer Tangente an eine Oberfläche des Haltelements veränderlich zu positionieren.

Vorzugsweise weist der vierte Führungsmechanismus ein Führungselement, wie z. B. eine Schienenführung, eine Teleskopführung oder eine Linearführung auf. Das Führungselement kann dazu ausgebildet sein, das Hilfselement entlang der Tangente an die Oberfläche des Haltelements zu positionieren. Die Tangente an die Oberfläche des Halteelements kann insbesondere parallel zu einer Z-Richtung der Lokalspule ausgerichtet sein. Eine Ausrichtung der Tangente and die Oberfläche des Haltelements kann mit einer Erstreckungsrichtung des Führungselements übereinstimmen.

Der vierte Führungsmechanismus kann weiterhin einen Führungsmechanismus gemäß einer Ausführungsform des dritten Führungsmechanismus umfassen. Dadurch kann zusätzlich eine Rotation des vierten Führungsmechanismus um eine Achse des Haltelements ermöglicht werden, wodurch sich das Hilfsmittel auf vorteilhafte Weise genauer relativ zu der diagnostisch relevanten Körperregion des Patienten positionieren lässt.

Vorzugsweise ist der vierte Führungsmechanismus dazu ausgebildet, das Hilfselement unabhängig von dem dritten Führungsmechanismus zu bewegen und/oder zu positionieren. In einer Ausführungsform sind der dritte Führungsmechanismus und der vierte Führungsmechanismus separat voneinander mit dem Halteelement verbunden.

Es ist vorstellbar, dass eine Ausrichtung der durch den vierten Führungsmechanismus definierten Tangente an die Oberfläche des Halteelements mittels einer mechanischen Verbindung zwischen dem vierten Führungsmechanismus und dem Haltelement fixiert ist. Beispielsweise kann der vierte Führungsmechanismus eine Schelle oder eine vergleichbare mechanische Verbindung umfassen, welche den vierten Führungsmechanismus in einer vorbestimmten Ausrichtung mit dem Halteelement verbindet.

Ein Hilfselement kann eine beliebige Vorrichtung darstellen, welche ein Erfassen von Magnetresonanzsignalen der diagnostisch relevanten Körperregion des Patienten ermöglicht und/oder unterstütz. In einer bevorzugten Ausführungsform umfasst das Hilfselement ein Spiegelelement, welches mittels des vierten Führungsmechanismus entlang einer Z-Richtung der Lokalspule veränderlich positionierbar ist.

Durch das Bereitstellen einer erfindungsgemäßen Lokalspule mit einem vierten Führungsmechanismus lässt sich ein Hilfselement auf vorteilhafte Weise mit dem Haltelement verbinden und unabhängig von einer Ausrichtung der zumindest einen Antenne relativ zu der diagnostisch relevanten Körperregion des Patienten positionieren. Ein Spiegelelement kann ein Sichtfeld des Patienten auf vorteilhafte Weise erweitern und insbesondere bei Kindern und/oder klaustrophobischen Patienten ein Risiko eines Abbruchs der Magnetresonanzmessung reduzieren.

In einer weiteren Ausführungsform der erfindungsgemäßen Lokalspule weist das Basiselement zwei Begrenzungselemente auf, welche einen von dem Basiselement definierten Aufnahmebereich von zwei einander gegenüberliegenden Seiten flankieren.

Vorzugsweise ist das Führungssystem, insbesondere der erste Führungsmechanismus und/oder der zweite Führungsmechanismus, mit genau einem Begrenzungselement gekoppelt oder mechanisch mit genau einem Begrenzungselement verbunden. Es ist insbesondere vorstellbar, dass das Führungssystem sowie das Halteelement von genau einem Begrenzungselement getragen werden. Das eine Begrenzungselement kann insbesondere als eine Trägerstruktur oder ein Trägerarm für das Führungssystem sowie das Halteelement ausgestaltet sein.

Erfindungsgemäß überschreitet eine Höhe eines ersten Begrenzungselements eine Höhe eines zweiten Begrenzungselements.

Es ist vorstellbar, dass das zweite Begrenzungselement und das Halteelement mit der zumindest einen Antenne durch einen Freiraum voneinander getrennt sind, sodass eine Kollision der zumindest eine Antenne mit dem zweiten Begrenzungselement beim Überführen der Lokalspule in die Schließposition vermieden wird.

Die zwei Begrenzungselemente des Basiselements können die diagnostisch relevante Körperregion des Patienten bei anwendungsgemäßer Positionierung des Patienten relativ zu der Lokalspule von zwei gegenüberliegenden Seiten flankieren oder zumindest teilweise umschließen.

Durch das Bereitstellen eines ersten Begrenzungselements lässt sich auf vorteilhafte Weise eine einseitige Haltestruktur für das Halteelement realisieren, sodass das zweite Ende des Halteelements freischwebend ausgestaltet sein kann. Ein niedrigeres zweites Begrenzungselement kann einen Freiraum oder Spalt zwischen dem Halteelement und einem nächstgelegenen Punkt des Basiselements vorteilhaft vergrößern, wodurch sich ein Zugang zu dem Aufnahmebereich und/oder eine Offenheit der Lokalspule gegenüber konventionellen Lokalspulen verbessern lässt.

In einer Ausführungsform der erfindungsgemäßen Lokalspule sind das zweite Begrenzungselement und das Halteelement durch einen Freiraum oder Spalt voneinander getrennt. Der Freiraum erstreckt sich entlang einer Flächennormalen einer durch das Basiselement definierten Auflagefläche in Richtung eines Aufnahmebereichs der Lokalspule.

Es ist vorstellbar, dass der Freiraum durch ein freies Volumen oder einen unverbauten Raum charakterisiert ist. Der Freiraum kann ebenso durch einen Spalt definiert sein, welcher das zweite Begrenzungselement und das Halteelement mit der zumindest einen Antenne voneinander trennt oder beabstandet. Vorzugsweise erstreckt sich der Freiraum über eine gesamte Abmessung des Basiselements in Z-Richtung der Lokalspule, über eine Breite des Spalts, welcher das Halteelement und die zumindest eine Antenne von dem zweiten Begrenzungselement trennt, sowie über eine gesamte Höhe der Lokalspule in einer Y-Richtung.

Durch das Bereitstellen einer erfindungsgemäßen Lokalspule mit einem Freiraum lassen sich ein Zugang zu dem Aufnahmebereich bzw. der diagnostisch relevanten Körperregion und/oder eine Offenheit der Lokalspule gegenüber konventionellen Lokalspulen auf vorteilhafte Weise verbessern. Dadurch lässt sich eine Effizienz und/oder eine Reproduzierbarkeit einer Positionierung der zumindest einen Antenne an der diagnostisch relevanten Körperregion des Patienten auf vorteilhafte Weise erhöhen.

Gemäß einer Ausführungsform der erfindungsgemäßen Lokalspule umfasst zumindest ein Begrenzungselement ein Griffelement.

Es ist vorstellbar, dass das erste Begrenzungselement und/oder das zweite Begrenzungselement ein Griffelement aufweisen. Vorzugsweise ist das Griffelement dazu ausgebildet, einen Transport der Lokalspule, insbesondere ein Anheben, Absetzen und/oder Ausrichten der Lokalspule, durch einen Nutzer zu ermöglichen.

Ein Griffelement kann beispielweise durch eine Aussparung in dem zumindest einen Begrenzungselement ausgeführt sein. Es ist jedoch ebenso vorstellbar, dass das Griffelement als ein dediziertes Griffstück, wie zum Beispiel ein Knauf, eine Stange oder ein Bügel, ausgestaltet ist.

Durch das Bereitstellen einer erfindungsgemäßen Lokalspule mit einem Griffelement kann auf vorteilhafte Weise auf zusätzliches Zubehör zur Handhabung und/oder zum Transport der Lokalspule verzichtet werden. Weiterhin kann durch das Vorsehen einer Aussparung in dem zumindest einen Begrenzungselement eine Offenheit der Lokalspule weiter verbessert werden.

In einer Ausführungsform weist die erfindungsgemäße Lokalspule ein Stützelement auf, welches in einer anwendungsgemäßen Position mit dem Basiselement verbunden ist und dazu ausgebildet ist, die in einem von dem Basiselement definierten Aufnahmebereich positionierte diagnostisch relevante Körperregion in einer vorbestimmten Position zu der Lokalspule zu stabilisieren.

Das Stützelement kann mittels einer mechanischen Verbindung mit dem Basiselement verbunden sein. Die mechanische Verbindung kann insbesondere als eine formschlüssige, eine kraftschlüssige und/oder eine stoffliche Verbindung ausgeführt sein.

Vorzugsweise weist das Stützelement eine Form auf, welche die diagnostisch relevante Körperregion in der vorbestimmten Position zu der Lokalspule stabilisiert. Dies kann bedeuten, dass das Stützelement dazu ausgebildet ist, eine Bewegung des Patienten, insbesondere der diagnostisch relevanten Körperregion, zu verhindern, zu behindern und/oder zu erschweren. Das Stützelement kann beispielsweise eine Vertiefung und/oder eine Aussparung aufweisen, welche dazu ausgebildet ist, die diagnostisch relevante Körperregion aufzunehmen. Eine Oberfläche des Stützelements kann entsprechend einer Kontur der diagnostisch relevanten Körperregion nachgeformt sein. Es ist ebenso vorstellbar, dass das Stützelement dazu ausgebildet ist, die diagnostisch relevante Körperregion an dedizierten Punkten zu stützen. In beispielhaften Ausführungen kann das Stützelement als eine Nackenrolle, eine U-förmiges Kissen oder eine Schalenförmiges Kissen ausgestaltet sein.

In einer Ausführungsform weist das Stützelement ein elastisches Material auf.

Beispiele für elastische Materialien sind Elastomere, insbesondere Kunststoffe auf Basis von Polyethen, Polyurethan, Polyamid und Polyester. Daneben sind auch Materialien auf natürlicher Basis, wie z. B. Kautschuk oder elastische Fasermaterialien, vorstellbar. In einer bevorzugten Ausführungsform ist das elastische Material ein Schaumstoff. Ein Stützelement mit einem elastischen Material kann auf vorteilhafte Weise ein geringes Gewicht aufweisen. Ferner kann sich ein Stützelement mit einem elastischen Material einer Form oder Oberflächenkontur der diagnostischen Körperregion des Patienten anpassen und dadurch eine Immobilisierung der diagnostisch relevanten Körperregion bewirken.

Das erste Begrenzungselement und das zweite Begrenzungselement sind dazu ausgebildet, das Stützelement mittels elastischer Rückstellkräfte des elastischen Materials in einer anwendungsgemäßen Position in der Lokalspule zu halten.

Das Stützelement kann derart mit dem Basiselement verbunden sein, dass das Stützelement durch eine Positionierung zwischen den zwei Begrenzungselementen elastisch verformt wird. Durch die elastische Verformung können elastische Rückstellkräfte in dem Stützelement aufgebaut werden, welche ein Verrutschen oder eine unbeabsichtigte Bewegung des Stützelements relativ zu dem Basiselement verhindern oder erschweren. Insbesondere können die zwei Begrenzungselemente in einem Winkel zueinander angeordnet sein, um eine formschlüssige Verbindung zwischen dem Stützelement und den zwei Begrenzungselementen bereitzustellen.

Durch eine erfindungsgemäße Lokalspule mit einem Stützelement lässt sich die diagnostisch relevante Körperregion auf vorteilhafte Weise immobilisieren (d. h. eine Bewegung der diagnostisch relevanten Körperregion des Patienten während einer Bildgebungsuntersuchung reduzieren), ohne ein Sichtfeld und/oder eine Gesichtsregion des Patienten zu beeinträchtigen.

In einer bevorzugten Ausführungsform der erfindungsgemäßen Lokalspule umfassen das Halteelement und/oder das Basiselement eine elektrische Schnittstelle, welche dazu ausgebildet ist, die zumindest eine Antenne mit einer Empfangseinheit einer Magnetresonanzvorrichtung zu verbinden.

Die elektrische Schnittstelle kann eine elektronische Schaltung umfassen. Die elektronische Schaltung kann ein elektronisches Bauelement oder einen Zusammenschluss von mehreren elektronischen Bauelementen, wie z. B. Transistoren, Widerständen, Kondensatoren, Dioden, Leiterbahnen und dergleichen, umfassen. Die elektronische Schaltung kann insbesondere eine Schutzschaltung aufweisen, welche dazu ausgebildet ist, die zumindest eine Antenne gegen eine Überlastung zu schützen. Für die Vermeidung magnetischer Anziehungskräfte, stehender Wellen, Aufheizung und vergleichbarer, unerwünschter Effekte, kann die elektronische Schaltung einen hohen Anteil nichtmagnetischer Materialien sowie entsprechende Mantelwellensperren und/oder Baluns aufweisen. Die elektronische Schaltung weist vorzugsweise eine Leiterplatte (PCB) oder ein vergleichbares Substrat auf, welches dazu geeignet ist, die elektronischen Bauelemente in einer vorbestimmten Position zueinander aufzunehmen.

In einer bevorzugten Ausführungsform weist die elektrische Schnittstelle einen ersten Anschluss. Es ist vorstellbar, dass die Empfangseinheit der Magnetresonanzvorrichtung mittels einer Signalleitung, beispielweise einer elektrischen Anschlussleitung, mit dem ersten Anschluss verbindbar ist. Die elektrische Schnittstelle kann ferner einen zweiten Anschluss aufweisen. Der zweite Anschluss kann mittels einer Signalleitung elektrisch mit der zumindest einen Antenne verbindbar sein. Vorzugsweise sind die zumindest eine Antenne und/oder die Empfangseinheit der Magnetresonanzvorrichtung reversibel mit der elektrischen Schnittstelle verbindbar. Eine reversible elektrische Verbindung kann beispielsweise durch ein Stecker-Buchsen-Prinzip bereitgestellt sein.

Durch das Bereitstellen einer erfindungsgemäßen Lokalspule mit einer elektrischen Schnittstelle kann auf eine direkte elektrische Anschlussleitung zwischen der zumindest einen Antenne und der Empfangseinheit auf vorteilhafte Weise verzichtet werden. Dadurch lässt sich ein mechanischer Widerstand der elektrischen Anschlussleitung, welcher eine Bewegung der zumindest einen Antenne stören kann, auf vorteilhafte Weise vermeiden. Weiterhin kann eine Beeinträchtigung des Patienten durch eine elektrische Anschlussleitung im Aufnahmebereich der Lokalspule vorteilhaft verhindert werden.

Die erfindungsgemäße Magnetresonanzvorrichtung umfasst eine Lokalspule gemäß einer oben beschriebenen Ausführungsform. In einer bevorzugten Ausführungsform ist die Lokalspule mechanisch mit einem Patiententisch und/oder einer Patientenlagerungsvorrichtung der Magnetresonanzvorrichtung verbunden. Die Magnetresonanzvorrichtung und/oder die Lokalspule können insbesondere eine Positionierungseinheit aufweisen, welche dazu ausgebildet ist, die Lokalspule relativ zu der Magnetresonanzvorrichtung, dem Patienten und/oder der Patientenlagerungsvorrichtung zu positionieren. Die Lokalspule umfasst zumindest eine Antenne, ein Basiselement, ein Halteelement und ein Führungssystem gemäß einer oben beschriebenen Ausführungsform. Die Magnetresonanzvorrichtung ist dazu ausgebildet, mittels der Lokalspule Magnetresonanzdaten einer diagnostisch relevanten Körperregion eines Patienten zu erfassen.

Ferner weist die erfindungsgemäße Magnetresonanzvorrichtung zumindest eine elektrische Anschlussleitung auf, welche dazu ausgelegt ist, die zumindest eine Antenne der Lokalspule elektrisch mit der Magnetresonanzvorrichtung, insbesondere einer Empfangseinheit der Magnetresonanzvorrichtung, zu verbinden.

In einer Ausführungsform weist die Lokalspule eine oder mehrere Sendeantennen auf, welche als eine Sendeeinheit ausgebildet sind. Die Sendeeinheit kann mittels einer elektrischen Anschlussleitung, z. B. über eine elektrische Schnittstelle gemäß einer oben beschriebenen Ausführungsform, mit einer Hochfrequenzeinheit der Magnetresonanzvorrichtung verbunden sein. Es ist vorstellbar, dass die Hochfrequenzeinheit einen Wechselstrom bereitstellt, welcher als hochfrequentes Signal von der Sendeeinheit in ein Volumen der diagnostisch relevanten Körperregion des Patienten ausgesendet wird, sodass ein B1-Magnetfeld erzeugt wird.

In einer weiteren Ausführungsform weist die Lokalspule eine oder mehrere Antennen auf, welche dazu ausgebildet sind, Magnetresonanzsignale der diagnostisch relevanten Körperregion des Patienten zu empfangen. Die zumindest eine Antenne kann mittels einer elektrischen Anschlussleitung mit einem Empfängerkanal der Magnetresonanzvorrichtung verbunden sein. Vorzugsweise ist die zumindest eine Antenne mittels einer elektrischen Anschlussleitung mit einer elektrischen Schnittstelle gemäß einer oben beschriebenen Ausführungsform verbunden. Die elektrische Schnittstelle kann dazu ausgebildet sein, ein von der zumindest einen Antenne empfangenes Magnetresonanzsignal mittels einer weiteren elektrischen Anschlussleitung bzw. Signalleitung and die Empfangseinheit der Magnetresonanzvorrichtung zu übertragen. Die Magnetresonanzvorrichtung ist somit in der Lage, Magnetresonanzsignale der diagnostisch relevanten Körperregion des Patienten zu empfangen und Magnetresonanzbilder in Abhängigkeit der empfangenen Magnetresonanzsignale zu rekonstruieren.

Durch die erfindungsgemäße Magnetresonanzvorrichtung lässt sich eine zeiteffiziente und reproduzierbare Aufnahme von Magnetresonanzbildern der Kieferregion des Patienten ermöglichen. Weiterhin teilt die erfindungsgemäße Magnetresonanzvorrichtung die Vorteile der erfindungsgemäßen Lokalspule gemäß einer oben beschriebenen Ausführungsform.

Weitere Vorteile und Einzelheiten ergeben sich aus der nachfolgenden Beschreibung von Ausführungsbeispielen in Verbindung mit den Zeichnungen. Es zeigen in Prinzipdarstellung:
Fig. 1 eine schematische Darstellung einer Ausführungsform einer erfindungsgemäßen Magnetresonanzvorrichtung,
Fig. 2 eine Darstellung einer Ausführungsform einer erfindungsgemäßen Lokalspule,
Fig. 3 eine Darstellung einer Ausführungsform einer erfindungsgemäßen Lokalspule,
Fig. 4 eine Darstellung einer Ausführungsform einer erfindungsgemäßen Lokalspule,
Fig. 5 eine Darstellung einer Ausführungsform einer erfindungsgemäßen Lokalspule,
Fig. 6 eine Darstellung einer Ausführungsform einer erfindungsgemäßen Lokalspule,
Fig. 7 eine Darstellung einer Ausführungsform einer erfindungsgemäßen Lokalspule,
Fig. 8 eine Darstellung einer Ausführungsform einer erfindungsgemäßen Lokalspule,
Fig. 9 eine Darstellung einer Ausführungsform einer erfindungsgemäßen Lokalspule.

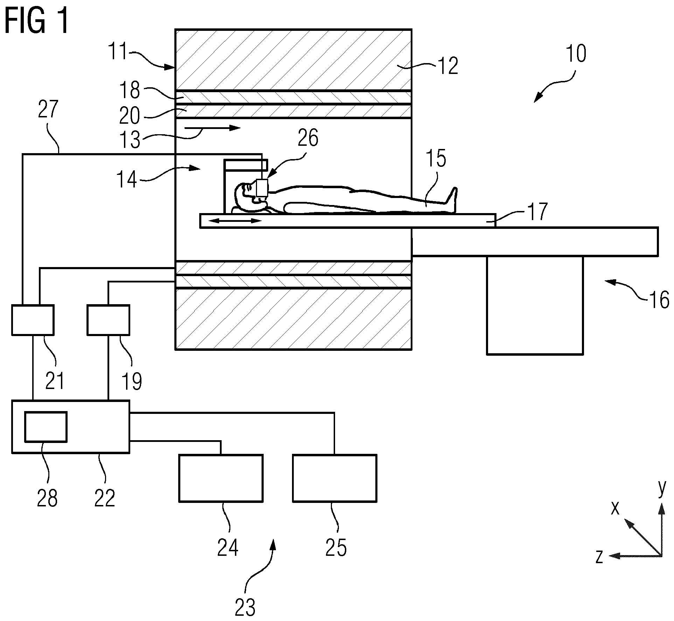

In Fig. 1 ist eine mögliche Ausführungsform einer erfindungsgemäßen Magnetresonanzvorrichtung 10 mit einer erfindungsgemäßen Lokalspule 26 schematisch dargestellt. Die Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines starken und insbesondere homogenen Grundmagnetfelds 13 (B0-Magnetfeld) aufweist. Zudem umfasst die Magnetresonanzvorrichtung 10 einen Patientenaufnahmebereich 14 zu einer Aufnahme eines Patienten 15. Der Patientenaufnahmebereich 14 ist in dem vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen der Magnetresonanzvorrichtung 10 und/oder des Patientenaufnahmebereichs 14 vorstellbar.

Die Magnetresonanzvorrichtung 10 weist vorzugsweise eine Patientenlagerungsvorrichtung 16 auf, welche dazu ausgebildet ist, den Patienten 15 in dem Patientenaufnahmebereich 14 zu positionieren. Die Patientenlagerungsvorrichtung 16 kann hierfür einen innerhalb des Patientenaufnahmebereichs 14 bewegbar ausgestalteten Patiententisch 17 umfassen.

In der in Fig. 1 gezeigten Ausführungsform weist die Magneteinheit 11 eine Gradientenspule 18 zum Erzeugen von magnetischen Gradientenfeldern auf, welche für eine Ortskodierung während einer Magnetresonanzmessung verwendet werden. Die Gradientenspule 18 wird mittels einer Gradientensteuereinheit 19 der Magnetresonanzvorrichtung 10 angesteuert. Die Magneteinheit 11 kann weiterhin eine Hochfrequenzantenne umfassen, welche in dem gezeigten Beispiel als fest in die Magnetresonanzvorrichtung 10 integrierte Körperspule 20 ausgestaltet ist. Die Körperspule 20 ist zu einer Anregung von Atomkernen ausgelegt, die sich in dem von dem Hauptmagneten 12 erzeugten Grundmagnetfeld 13 befinden. Die Körperspule 20 wird von einer Hochfrequenzeinheit 21 der Magnetresonanzvorrichtung 10 angesteuert und strahlt hochfrequente Signale in einen Untersuchungsraum ein, der im Wesentlichen von einem Patientenaufnahmebereich 14 der Magnetresonanzvorrichtung 10 gebildet ist. Die Körperspule 20 kann weiterhin auch zu einem Empfangen von Magnetresonanzsignalen ausgebildet sein.

Die Magnetresonanzvorrichtung 10 weist vorliegend eine Steuereinheit 22 auf, welche dazu ausgebildet ist, den Hauptmagneten 12, die Gradientensteuereinheit 19 und die Hochfrequenzeinheit 21 zu steuern und/oder zu koordinieren. Die Steuereinheit 22 ist vorzugsweise dazu ausgebildet eine Durchführung einer Sequenz, wie z. B. einer bildgebenden Gradientenechosequenz, einer TSE-Sequenz oder einer UTE-Sequenz, zu steuern. Die Steuereinheit 22 kann eine Auswerteeinheit 28 umfassen, welche zu einer Auswertung von digitalisierten Magnetresonanzsignalen, die während einer Magnetresonanzmessung erfasst werden, ausgestaltet ist.

Des Weiteren kann die Magnetresonanzvorrichtung 10 eine Benutzerschnittstelle 23 umfassen, welche eine Signalverbindung mit der Steuereinheit 22 aufweist. Steuerinformationen, wie beispielsweise Bildgebungsparameter und rekonstruierte Magnetresonanzbilder, können auf einer Anzeigeeinheit 24, beispielsweise auf zumindest einem Monitor der Benutzerschnittstelle 23, für einen Nutzer angezeigt werden. Weiterhin weist die Benutzerschnittstelle 23 eine Eingabeeinheit 25 auf, mittels der Parameter einer Magnetresonanzbildgebung von dem Nutzer eingegeben werden können.

Die Magnetresonanzvorrichtung 10 umfasst eine erfindungsgemäße Lokalspule 26, welche vorliegend an einem Kopf oder einer Kieferregion des Patienten 15 positioniert ist. Die Lokalspule 26 ist dazu ausgebildet, Magnetresonanzsignale aus einem Volumen der Kieferregion zu erfassen und an die Magnetresonanzvorrichtung 10 zu übertragen. Die Lokalspule 26 weist vorzugsweise eine elektrische Anschlussleitung 27 auf, welche eine Signalverbindung zwischen einer Antenne 32 der Lokalspule und einer Empfangseinheit der Magnetresonanzvorrichtung 10, insbesondere der Hochfrequenzeinheit 21 und/oder der Steuereinheit 22, bereitstellt.

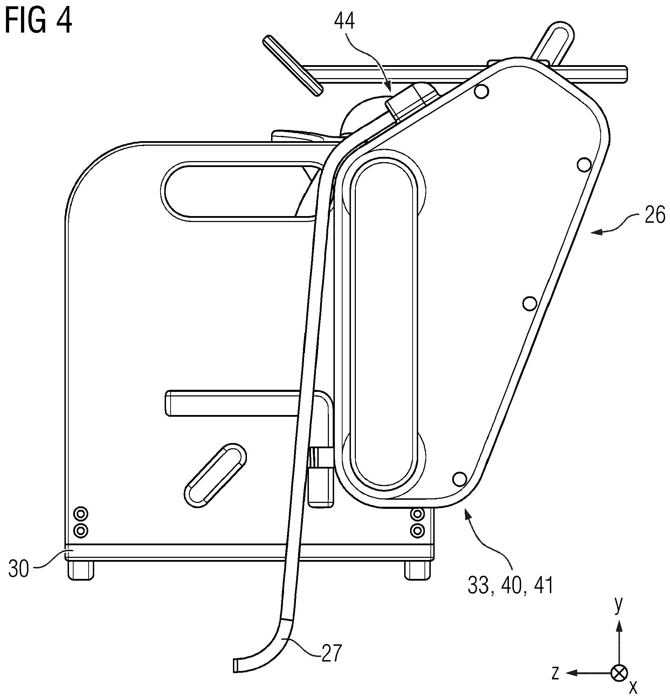

Eine oder mehrere Antennen 32 der Lokalspule 26 können auch elektrisch mit einer elektrischen Schnittstelle 44 (siehe Fig. 4) der Lokalspule 26 verbunden sein. Die elektrische Schnittstelle 44 kann wiederum mittels der gezeigten elektrischen Anschlussleitung 27 (siehe Fig. 1) mit der Empfangseinheit der Magnetresonanzvorrichtung 10 verbunden sein. Alternativ kann die Lokalspule 26 mittels einer drahtlosen Signalverbindung mit der Magnetresonanzvorrichtung 10 verbunden sein.

Analog zu der Körperspule 20 kann auch die Lokalspule 26 zu einer Anregung von Atomkernen und zu einem Empfangen von Magnetresonanzsignalen ausgebildet sein. Beispielweise wird zum Aussenden von hochfrequenten Signalen eine Sendeeinheit der Lokalspule 26 von der Hochfrequenzeinheit 21 angesteuert.

Die dargestellte Magnetresonanzvorrichtung 10 kann selbstverständlich weitere Komponenten umfassen, welche Magnetresonanzvorrichtungen üblicherweise aufweisen. Es ist ebenso vorstellbar, dass die Magnetresonanzvorrichtung 10 statt des zylinderförmigen Aufbaus einen C-förmigen, einen dreieckigen oder einen asymmetrischen Aufbau der Magnetfeld-erzeugenden Komponenten aufweist. Die Magnetresonanzvorrichtung 10 kann insbesondere eine dedizierte Magnetresonanzvorrichtung 10 sein, welche dazu ausgebildet ist, eine Magnetresonanzbildgebung der Kieferregion eines stehenden oder sitzenden Patienten 15 durchzuführen.

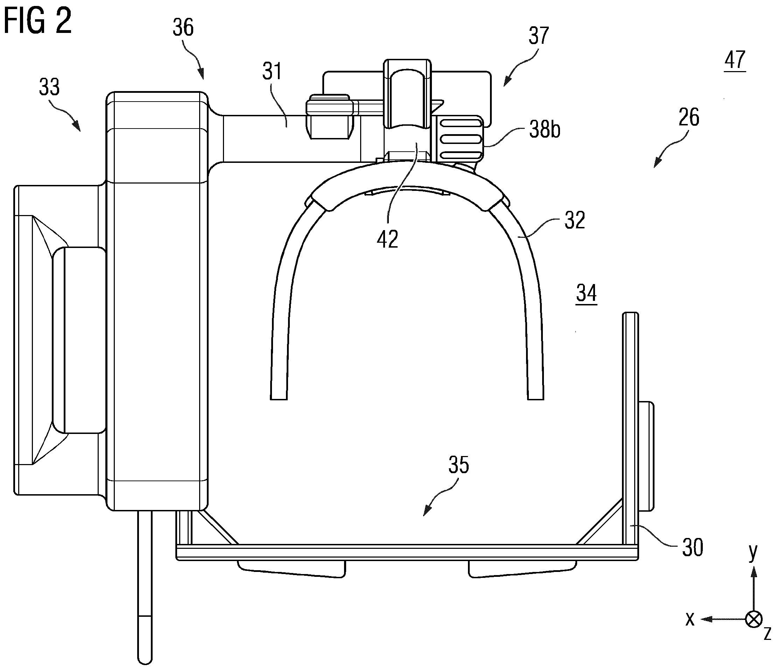

Fig. 2 zeigt eine beispielhafte Ausführungsform der erfindungsgemäßen Lokalspule 26. Eine oder mehrere Antennen 32 der Lokalspule 26 sind in einer Trägerstruktur eingebettet und werden von einem Halteelement 31 getragen. Das Haltelement 31 ist mittels des Führungssystems 33 relativ zu dem Basiselement 30 positionierbar. Eine oder mehrere der Antennen 32 können eine Sendeeinheit und/oder eine Empfangseinheit der Lokalspule 26 bilden. Das Halteelement 31 ist vorliegend als ein Arm mit einem im Wesentlichen zylindrischen Körper ausgeführt und an dem ersten Ende 36 mechanisch mit dem Führungssystem 33 bzw. dem Basiselement 30 verbunden. Das dem ersten Ende 36 gegenüberliegende zweite Ende 37 des Haltelements 31 ist freischwebend ausgestaltet. Dadurch wird ein Freiraum 34 bereitgestellt, welcher einen Zugang zu einer in dem Aufnahmebereich 35 positionierten Körperregion (nicht gezeigt) erleichtert. Gleichermaßen kann eine Beeinträchtigung eines in dem Aufnahmebereich 35 positionierten Patient 15 im Vergleich zu konventionellen Lokalspulen reduziert werden.

In der gezeigten Ausführungsform weist die Lokalspule 26 einen Führungsmechanismus 42 auf, welcher dazu ausgebildet ist, eine Rotation der Antenne 32 um eine Achse des Führungsmechanismus 42 zu ermöglichen. Der Führungsmechanismus 42 ist in dem gezeigten Beispiel rotierbar um einen zylindrischen Abschnitt des Halteelements 31 gelagert und ermöglicht eine Rotation der Antenne 32 um die X-Achse der Lokalspule 26.

Weiterhin weist die Lokalspule 26 in dem gezeigten Beispiel einen Arretierungsmechanismus 38b auf, welcher dazu ausgebildet ist, die Antenne 32 in einer gewünschten Rotationsstellung gegenüber dem Haltelement 31 festzustellen und/oder einen mechanischen Widerstand des Führungsmechanismus 42 einzustellen. Das Arretierungselement 38b weist vorliegend eine Stellschraube auf, welche es einem Nutzer ermöglicht, den mechanischen Widerstand des Führungsmechanismus 42 einzustellen.

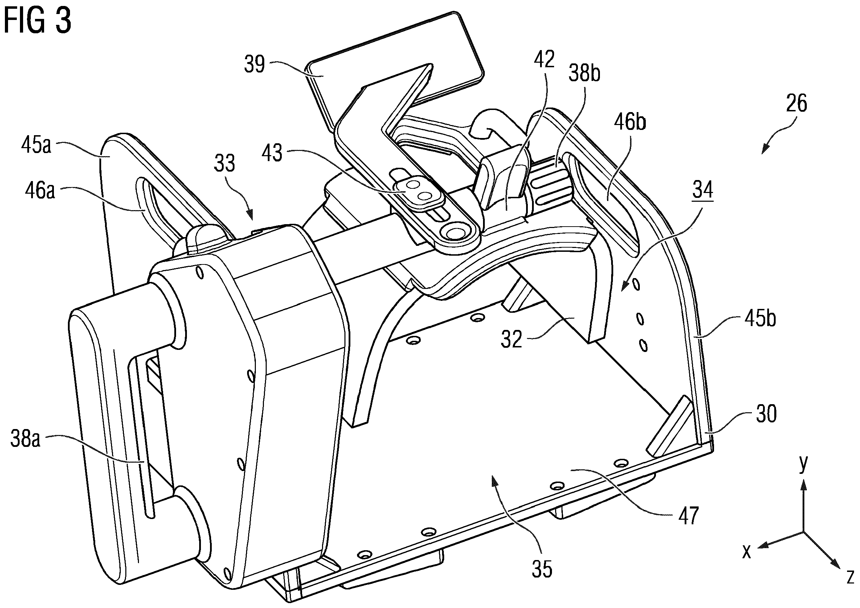

Fig. 3 zeigt eine Ausführungsform, in welcher die Lokalspule 26 einen Führungsmechanismus 43 aufweist. Der Führungsmechanismus 43 ist dazu ausgebildet, ein Hilfselement 39 veränderlich entlang einer Tangente an einer Oberfläche des Haltelements 31 zu positionieren. Vorzugsweise ist der Führungsmechanismus 43 dazu ausgebildet das Hilfselement 39 entlang einer Z-Richtung der Lokalspule 26 zu positionieren.

Der Führungsmechanismus 43 kann in einer vorbestimmten Ausrichtung mit dem Haltelement 31 mechanisch verbunden sein. Es ist jedoch ebenso vorstellbar, dass der Führungsmechanismus 43 analog zu dem Führungsmechanismus 42 ein Lager (nicht gezeigt) aufweist, welches eine Rotation des Hilfselements 39 um eine Achse oder einen Umfang des zylindrischen Körpers des Halteelements 31 ermöglicht. Vorzugsweise sind der Führungsmechanismus 412und der Führungsmechanismus 43 mechanisch unabhängig voneinander ausgestaltet, sodass die Antenne 32 und das Hilfselement 39 unabhängig voneinander positionierbar sind.

Das Basiselement 30 der in Fig. 3 gezeigten Lokalspule 26 besitzt zwei Begrenzungselemente 45a und 45b (45a-b), welche den von dem Basiselement 30 definierten oder eingefassten Aufnahmebereich 35 von zwei einander gegenüberliegenden Seiten flankieren. Ausgehend von der Auflagefläche 47 überschreitet die Höhe des ersten Begrenzungselements 45a die Höhe des zweiten Begrenzungselements 45b, sodass eine Kollision des Halteelements 31 und/oder der Antenne 32 mit dem zweiten Begrenzungselement 45b beim Überführen der Lokalspule 26 in die gezeigte Schließposition vermieden wird und ein zusätzlicher Freiraum bereitgestellt wird, welcher einen Zugang zu dem Aufnahmebereich 35 aus einem Umfeld 47 der Lokalspule 26 erleichtert. Die Begrenzungselemente 45a-b können Griffelemente 46a und 46b aufweisen, um einen Transport und/oder eine Ausrichtung der Lokalspule 26 relativ zu der Patientenlagerungsvorrichtung 16 und/oder dem Patiententisch 17 durch einen Nutzer zu ermöglichen.

In einer Ausführungsform kann die Lokalspule 26 ein Stützelement (nicht gezeigt) aufweisen, welches anwendungsgemäß auf der Auflagefläche 47 in dem Aufnahmebereich 35 positioniert ist. Vorzugsweise umfasst das Stützelement ein elastisches Material. Eine Abmessung des Stützelements in der X-Richtung kann eine Abmessung des Aufnahmebereichs 35 in der X-Richtung überschreiten, sodass das Stützelement zwischen den Begrenzungselementen 45a-b eingeklemmt und mittels elastischer Rückstellkräfte des elastischen Materials in der anwendungsgemäßen Position gehalten wird. Die Begrenzungselemente 45a-b können insbesondere unter einem Winkel zueinander angeordnet sein, sodass die freien Enden der Begrenzungselemente 45a-b einen geringeren Abstand zueinander aufweisen als die mit dem Basiselement 30 verbundenen Enden der Begrenzungselemente 45a-b. Dadurch kann ein Einklemmen des Stützelements zwischen den Begrenzungselementen 45a-b verbessert werden.

Fig. 4 zeigt eine Ausführungsform der erfindungsgemäßen Lokalspule 26 mit einer elektrischen Schnittstelle 44. Die Empfangseinheit der Magnetresonanzvorrichtung 10 kann mittels der elektrischen Anschlussleitung 27 mit einem ersten Anschluss der elektrischen Schnittstelle 44 verbunden sein, während die Antenne 32 mit einem zweiten Anschluss der elektrischen Schnittstelle 44 (nicht gezeigt) verbunden ist. Vorzugsweise ist der zweite Anschluss der elektrischen Schnittstelle 44 in das Haltelement 31 integriert, sodass eine Führung von Kabeln (oder elektrischen Leitungen) durch ein Volumen des Aufnahmebereichs 35 zwischen dem Basiselement 30 und dem Halteelement 31 vermieden wird.

In Fig. 4 liegt die erfindungsgemäße Lokalspule 26 in einer Schließposition vor, d. h. die Antenne 32 ist mittels des Führungsmechanismus 41 des Führungssystems 33 in Richtung der Auflagefläche 47 (siehe Fig. 3) der Lokalspule 26 ausgelenkt. Der Führungsmechanismus 41 ist vorliegend dazu ausgebildet, das Haltelement 31 veränderlich entlang der Y-Richtung der Lokalspule 26 zu positionieren.

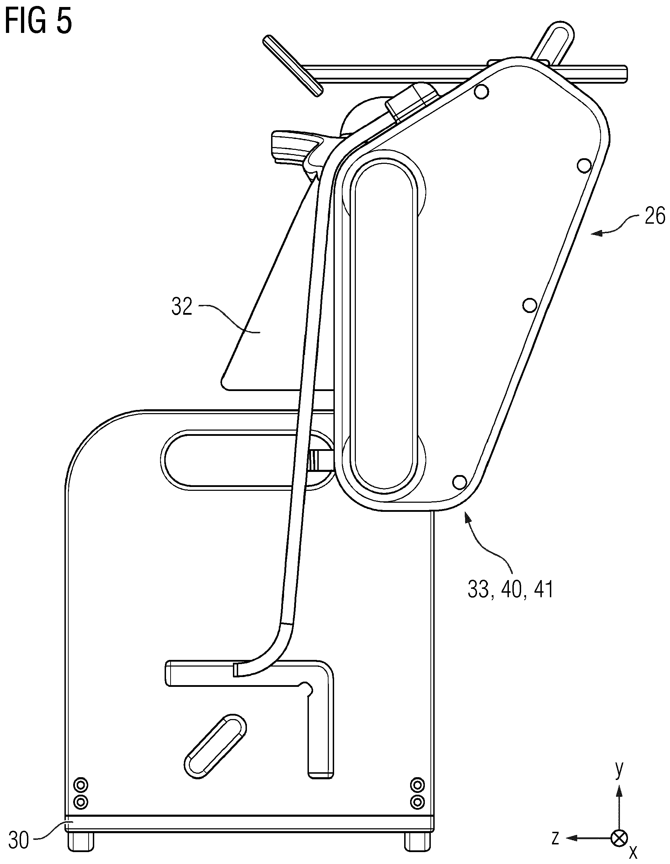

In Fig. 5 liegt die erfindungsgemäße Lokalspule 26 dagegen in einer Öffnungsposition vor, d. h. die Antenne 32 ist mittels des Führungsmechanismus 41 des Führungssystems 33 in einer der Auflagefläche 47 des Basiselements 30 entgegengesetzten Richtung ausgelenkt. Der Führungsmechanismus 41 kann insbesondere ein zweiter Führungsmechanismus gemäß einer oben beschriebenen Ausführungsform sein.

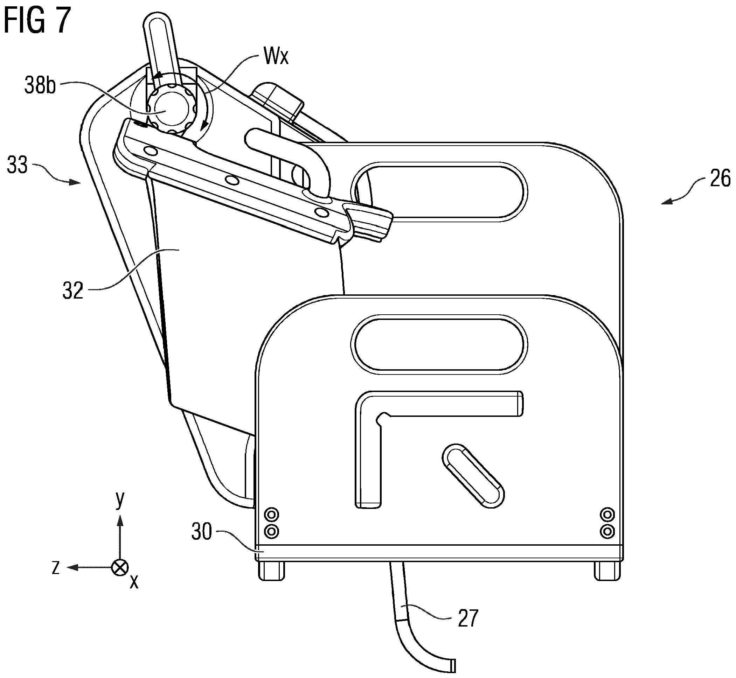

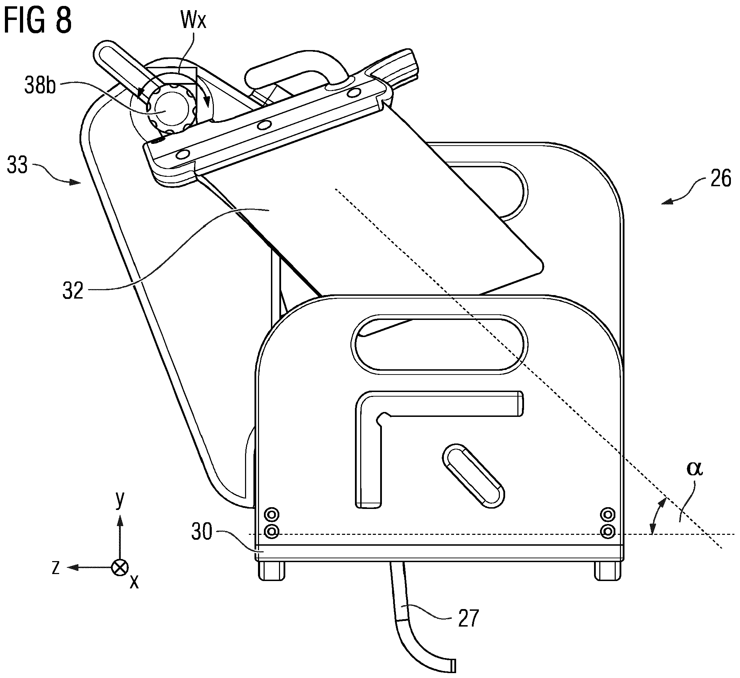

In einer bevorzugten Ausführungsform der erfindungsgemäßen Lokalspule 26 ist eine Überführung der Lokalspule 26 von der Schließposition in die Öffnungsposition erst dann möglich, wenn die zumindest eine Antenne 32, wie in den Figuren 7 und 8 gezeigt, mittels des Führungsmechanismus 42 von einer Messposition in eine Ausgangsposition überführt wurde. In der Messposition kann die Antenne 32 im Wesentlichen in Y-Richtung und/oder in Richtung des Aufnahmebereichs 35 ausgerichtet sein (vgl. Fig. 7). In der Ausgangsposition kann die Antenne 32 dagegen zumindest teilweise in Z-Richtung, z. B. mit einer Neigung unter einem Winkel α, ausgerichtet sein (vgl. Fig. 8).

Es ist vorstellbar, dass die Lokalspule 26 einen Sicherungsmechanismus aufweist, welcher ein Überführen der Lokalspule 26 von der Schließposition in die Öffnungsposition oder umgekehrt verhindert, wenn eine Neigung der Antenne 32 zu der Z-Achse oder der Auflagefläche 47 einen vorbestimmten Winkel α, z. B. 30° oder 40° oder 50°, unterschreitet.

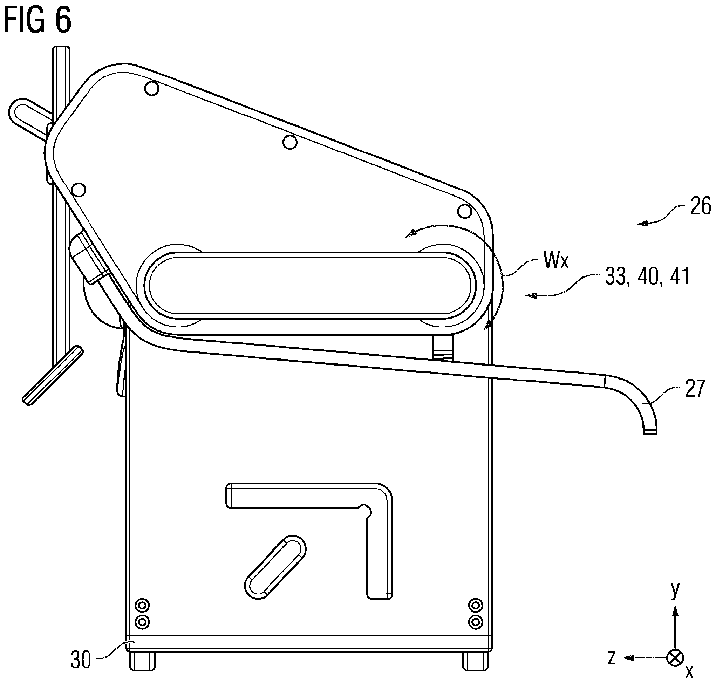

Fig. 6 zeigt eine Ausführungsform der erfindungsgemäßen Lokalspule 26, bei welcher das Halteelement 31 und die Antenne 32 mittels des Führungsmechanismus 40 ausgehend von der in Fig. 5 gezeigten Öffnungsposition entlang einer Bogenbahn in eine zweite Öffnungsposition überführt wurden. Die Bogenbahn kann dabei durch eine Bewegungstrajektorie des Halteelements 31 und/oder der Antenne 32 charakterisiert sein, welche sich durch eine Rotation des Führungsmechanismus 40 um die Drehrichtung Wx ergibt. Der Führungsmechanismus kann insbesondere ein erster Führungsmechanismus gemäß einer oben beschriebenen Ausführungsform sein.

In einer bevorzugten Ausführungsform der Lokalspule 26 sind der Führungsmechanismus 40 und der Führungsmechanismus 41 des Führungssystems 33 mechanisch gekoppelt oder integriert. Dies kann bedeuten, dass das Halteelement 31 mit der Antenne 32 beim Überführen der Lokalspule 26 von der Schließposition in die Öffnungsposition (oder umgekehrt) mittels der Führungsmechanismen 40 und 41 sowohl entlang der Y-Richtung als auch entlang der Drehrichtung Wx bewegt wird. Es ist ebenso vorstellbar, dass das Halteelement 31 beim Überführen der Lokalspule 26 von der Schließposition in die Öffnungsposition (in umgekehrt) zunächst entlang einer durch den Führungsmechanismus 41 vorgegebenen Bewegungstrajektorie und anschließend entlang einer durch den Führungsmechanismus 40 vorgegebenen Bewegungstrajektorie positioniert wird. Die Bewegungstrajektorie des Haltelements 31 kann dabei durch eine Bogenbahn charakterisiert sein, welche im Wesentlichen einem Abschnitt einer Ellipsenbahn entspricht.

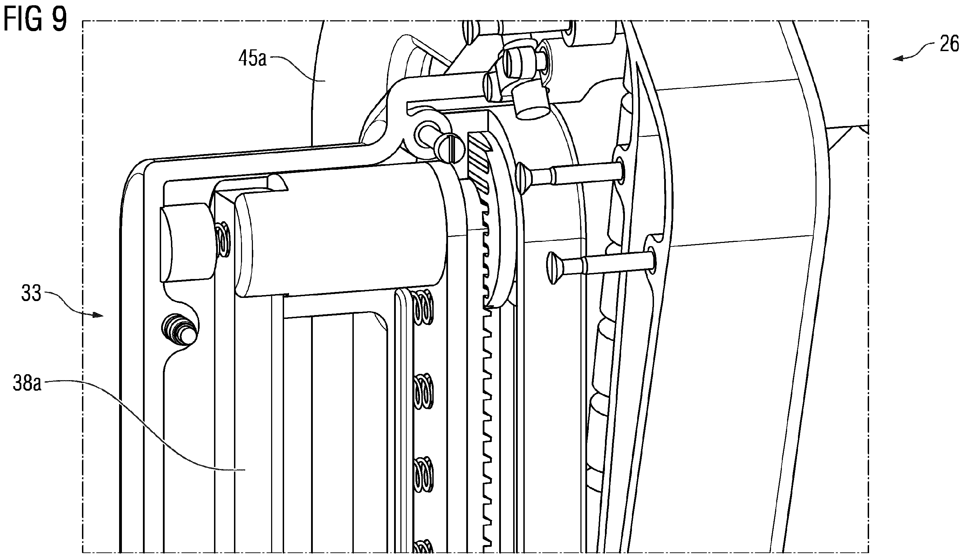

Fig. 9 zeigt eine Ausführungsform, in welcher die erfindungsgemäße Lokalspule 26 einen Arretierungsmechanismus 38a aufweist. Der Arretierungsmechanismus 38a ist vorliegend dazu ausgebildet, ein Positionieren des Halteelements 31 mittels des Führungsmechanismus 40 freizugeben oder den Führungsmechanismus 40 in einer gewünschten Position zu fixieren. Der Arretierungsmechanismus 38a umfasst ein an Federelementen gelagertes Rastelement, welches mittels manueller Krafteinwirkung durch einen Nutzer von einem komplementär ausgebildeten Rastelement des Führungssystems 33, insbesondere des Führungsmechanismus 40, gelöst werden kann. Durch einen Eingriff des Rastelements mit dem komplementären Rastelement des Führungsmechanismus 40 lassen sich der Führungsmechanismus 40 und der Führungsmechanismus 41 mechanisch koppeln, wodurch das Halteelement 31 bei einer Betätigung des Führungssystems 33 entlang einer Bewegungstrajektorie, welche sich aus einer Überlagerung der Bewegungstrajektorien der Führungsmechanismen 40 und 41 ergibt, geführt werden kann (z. B. entlang einer Bogenbahn oder einer Ellipsenbahn). Der Führungsmechanismus 40 ist in diesem Beispiel zur Führung des Halteelements 31 in einer Ausgangs-Position des Arretierungsmechanismus 38a freigegeben. Der Führungsmechanismus 40 kann durch eine Betätigung des Arretierungsmechanismus 38a von dem Führungsmechanismus 41 entkoppelt werden, sodass eine Bewegung des Haltelements 31 mittels des Führungssystems 33 auf eine durch den Führungsmechanismus 41 definierte Bewegungstrajektorie beschränkt wird (z. B. auf eine Gerade entlang der Y-Richtung).

Neben der in Fig. 9 gezeigten Ausführungsform sind selbstverständlich Abwandlungen der Ausgestaltung des Arretierungsmechanismus 38a vorstellbar. Beispielsweise kann der Arretierungsmechanismus 38a dazu ausgebildet sein, den Führungsmechanismus 40 und/oder den Führungsmechanismus 41 des Führungssystems 33 in Abhängigkeit einer Krafteinwirkung durch den Nutzer freizugeben. Der Arretierungsmechanismus 38a kann weiterhin ein Adaptionselement (nicht gezeigt) aufweisen, welches dazu ausgebildet ist, einen mechanischen Widerstand des Führungssystems 33, insbesondere des Führungsmechanismus 40 und/oder des Führungsmechanismus 41, veränderlich einzustellen.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Lokalspule (26) für eine Magnetresonanzvorrichtung (10), umfassend zumindest eine Antenne (32), ein Basiselement (30), ein Halteelement (31) und ein Führungssystem (33) wobei die zumindest eine Antenne (32) dazu ausgebildet ist, hochfrequente Signale in einem Frequenz- und Leistungsbereich einer Magnetresonanzmessung zu empfangen und wobei die zumindest eine Antenne (32) mechanisch mit dem Halteelement (31) verbunden ist,
wobei das Haltelement (31) dazu ausgebildet ist, die zumindest eine Antenne (32) in einer anwendungsgemäßen Position an einer diagnostisch relevanten Körperregion eines Patienten (15) zu halten, wobei das Führungssystem (33) mechanisch mit dem Basiselement (30) und dem Halteelement (31) verbunden ist und dazu ausgebildet ist, das Halteelement (31) veränderlich gegenüber dem Basiselement (30) zu positionieren,
wobei ein erstes Ende (36) des Halteelements (31) mechanisch mit dem Basiselement (30) verbunden ist und wobei ein dem ersten Ende gegenüberliegendes zweites Ende (37) des Halteelements (31) freischwebend ist.

2. Lokalspule (26) nach Anspruch 1, wobei das Führungssystem (33) einen ersten Führungsmechanismus (40) aufweist, welcher dazu ausgebildet ist, das Halteelement (31) relativ zu dem Basiselement (30) entlang einer Bogenbahn zu führen.

3. Lokalspule (26) nach einem der vorhergehenden Ansprüche, wobei das Führungssystem (33) einen zweiten Führungsmechanismus (41) aufweist, welcher dazu ausgebildet ist, das Halteelement (31) im Wesentlichen entlang einer Flächennormalen einer von dem Basiselement (30) definierten Auflagefläche (47) veränderlich zu positionieren.

4. Lokalspule (26) nach einem der vorhergehenden Ansprüche, aufweisend einen Arretierungsmechanismus (38a), welcher dazu ausgebildet ist,
- ein Positionieren des Halteelements (31) mittels des Führungssystems (33) freizugeben und/oder
- das Führungssystem (33) in einer gewünschten Position zu fixieren.

5. Lokalspule (26) nach Anspruch 4, wobei der Arretierungsmechanismus (38a) ein Adaptionselement aufweist, welches dazu ausgebildet ist, einen mechanischen Widerstand des Führungssystems (33) veränderlich einzustellen.

6. Lokalspule (26) nach einem der vorhergehenden Ansprüche, aufweisend einen dritten Führungsmechanismus (42), welcher dazu ausgebildet ist, eine Rotation der zumindest einen Antenne (32) um eine Achse des dritten Führungsmechanismus (42) zu ermöglichen.

7. Lokalspule (26) nach Anspruch 6, wobei das Haltelement (31) einen zylindrischen Körper aufweist und wobei der dritte Führungsmechanismus (42) dazu ausgebildet ist, eine Rotation der zumindest einen Antenne (32) um eine Achse des zylindrischen Körpers des Haltelements (31) zu ermöglichen.

8. Lokalspule (26) nach einem der Ansprüche 6 oder 7, ferner aufweisend einen zweiten Arretierungsmechanismus (38b), welcher dazu ausgebildet ist, eine Rotation der zumindest einen Antenne (32) um die Achse des dritten Führungsmechanismus (42) freizugeben und/oder den dritten Führungsmechanismus (42) in einer gewünschten Position zu fixieren.

9. Lokalspule (26) nach einem der vorhergehenden Ansprüche, aufweisend einen vierten Führungsmechanismus (43), welcher mechanisch mit dem Haltelement (31) verbunden ist und dazu ausgebildet ist, ein Hilfselement (39) entlang einer Tangente an eine Oberfläche des Haltelements (31) veränderlich zu positionieren.

10. Lokalspule (26) nach einem der vorhergehenden Ansprüche, wobei das Basiselement zwei Begrenzungselemente (45) aufweist, welche einen von dem Basiselement (30) definierten Aufnahmebereich (35) von zwei einander gegenüberliegenden Seiten flankieren, wobei eine Höhe eines ersten Begrenzungselements (45a) eine Höhe eines zweiten Begrenzungselements (45b) überschreitet.

11. Lokalspule (26) nach Anspruch 10, wobei das zweite Begrenzungselement (45b) und das Halteelement (31) durch einen Freiraum (34) voneinander getrennt sind, wobei sich der Freiraum (34) entlang einer Flächennormalen einer durch das Basiselement (30) definierten Auflagefläche (37) in Richtung eines Aufnahmebereichs (35) der Lokalspule (26) erstreckt.

12. Lokalspule (26) nach einem der Ansprüche 10 oder 11, wobei zumindest ein Begrenzungselement (45) ein Griffelement (46) umfasst.

13. Lokalspule (26) nach einem der vorhergehenden Ansprüche, ferner aufweisend ein Stützelement, welches in einer anwendungsgemäßen Position mit dem Basiselement (30) verbunden ist und dazu ausgebildet ist, die in einem Aufnahmebereich (35) der Lokalspule (26) positionierte diagnostisch relevante Körperregion in einer vorbestimmten Position zu der Lokalspule (26) zu stabilisieren.

14. Lokalspule (26) nach einem der Ansprüche 10 bis 12 und Anspruch 13, wobei das Stützelement ein elastisches Material aufweist und wobei das erste Begrenzungselement (45a) und das zweite Begrenzungselement (45b) dazu ausgebildet sind, das Stützelement mittels elastischer Rückstellkräfte des elastischen Materials in einer anwendungsgemäßen Position in der Lokalspule (26) zu halten.

15. Lokalspule (26) nach einem der vorhergehenden Ansprüche, wobei das Halteelement (31) und/oder das Basiselement (30) eine elektrische Schnittstelle (44) umfassen, welche dazu ausgebildet ist, die zumindest eine Antenne (32) mit einer Empfangseinheit einer Magnetresonanzvorrichtung (10) zu verbinden.

16. Magnetresonanzvorrichtung (10), umfassend eine Lokalspule (26) nach einem der vorhergehenden Ansprüche, wobei die Magnetresonanzvorrichtung (10) dazu ausgebildet ist, mittels der Lokalspule (26) Magnetresonanzdaten der diagnostisch relevanten Körperregion des Patienten (15) zu erfassen.
